# EUROPEAN PATENT SPECIFICATION

(11) **EP 1 728 534 B1**
(45) Date of publication and mention of the grant of the patent: **19.08.2015**
(21) Application number: 06114460.6
(22) Date of filing: 24.05.2006
(51) Int. Cl.: A61N 5/06

(54) **Skin treatment unit**
Hautbehandlungsvorrichtung
Dispositif pour le traitement de la peau

(30) Priority: 01.06.2005 IT BO20050382
(43) Date of publication of application: 06.12.2006
(73) Proprietor: Espansione Marketing S.p.A., 40050 Argelato, Frazione Funo (BO) (IT)
(72) Inventor: Pomar, Rodolfo, 40067, Pianoro, Frazione Rastignano BO (IT)
(74) Representative: Manzella & Associati

(56) References cited:
- WO-A-02/062420
- WO-A-03/043514
- WO-A-03/047682
- WO-A-03/081127
- WO-A-2004/096072
- WO-A-2006/012752
- DE-A1-102004 063 370
- US-A1- 2004 167 497

## Description

The present invention relates to a radiation treatment unit for treating the skin for aesthetic or therapeutic purposes.

It is known that by subjecting the derma of a person to appropriate cycles of exposure to light beams of preset wavelengths it is possible to treat certain blemishes and contrast certain problems of the skin.

The design choice that entails the greatest advantages in skin treatment provides for the use of small assemblies (provided with a light source), which can be directed toward a small portion of the derma and arranged at short distances from it.

Among these, the constructive solution disclosed in EP 1535582 in the name of the same Applicant and entitled "Light irradiation unit" offers the extremely advantageous possibility to substantially replace the source (or a filter interposed between the source and the skin), using for each type of skin the light source that is most suited in combination with an appropriate treatment cycle.

The diffusion of light emitting diodes and the corresponding extension of their possible fields of application have recently led some manufacturers to study skin treatment devices that use diodes as light sources.

Among the advantages provided by diodes with respect to other sources, besides the fact that they have the first faults linked to aging after extremely longer periods than other light sources, one has to consider the extreme precision of the wavelength of the emitted beam: this entails first of all the possibility to not use any filter to eliminate dangerous frequencies and secondly a great specificity of each treatment.

This type of apparatus is constituted by a basic supporting structure, with respect to which a sort of screen can move and be oriented, the surface of said screen comprising a distributed plurality of light emitting diodes.

The treatment is performed by moving the screen close to the face (or to the part to be treated) and by starting a cycle of emissions.

The surface of the face (in particular) and of other parts of the body that can benefit from a treatment of this type, however, are extremely irregular. Consider in particular the face: the nose protrudes and some regions are in shadow while others are perfectly illuminated, and therefore it is normal for the treatment to be rather uneven.

Every known type of apparatus is adapted to irradiate the treated region with a light beam of a certain wavelength which depends on the characteristics of the installed diodes: to perform different treatments it is necessary to have available different apparatuses, each of which emits a stream of a certain wavelength.

An important problem that arises from the possibility to use devices having different characteristics, in addition to the space occupation and installation, is linked to the cost, which for devices of this type is extremely high and makes it almost prohibitive to have an apparatus for each wavelength of interest.

From documents WO 2003/043514, WO 02/062420 and WO 2004/096072; examples of light radiation treatment devices are available.

The aim of the present invention is to obviate the cited drawbacks and meet the mentioned requirements, by providing a cartridge for skin treatment, with light beams emitted by light-emitting diodes, which is compact and suitable for the uniform treatment of delimited areas.

Within this aim, an object of the present invention is to provide a series of interchangeable cartridges, each of which emits, by means of diodes, a light beam having a certain wavelength, said cartridges being adapted to be installed in an appropriate skin treatment unit.

Another object of the present invention is to provide a skin treatment cartridge which is simple, relatively easy to provide in practice, safe in use, effective in operation, and has a relatively low cost.

This aim and these objects are achieved by the present skin treatment unit according to the invention, that has the features set forth in claim 1.

Further characteristics and advantages of the invention will become better apparent from the following detailed description of a preferred but not exclusive embodiment of a skin treatment cartridge, illustrated by way of non-limiting example in the accompanying drawings, wherein:

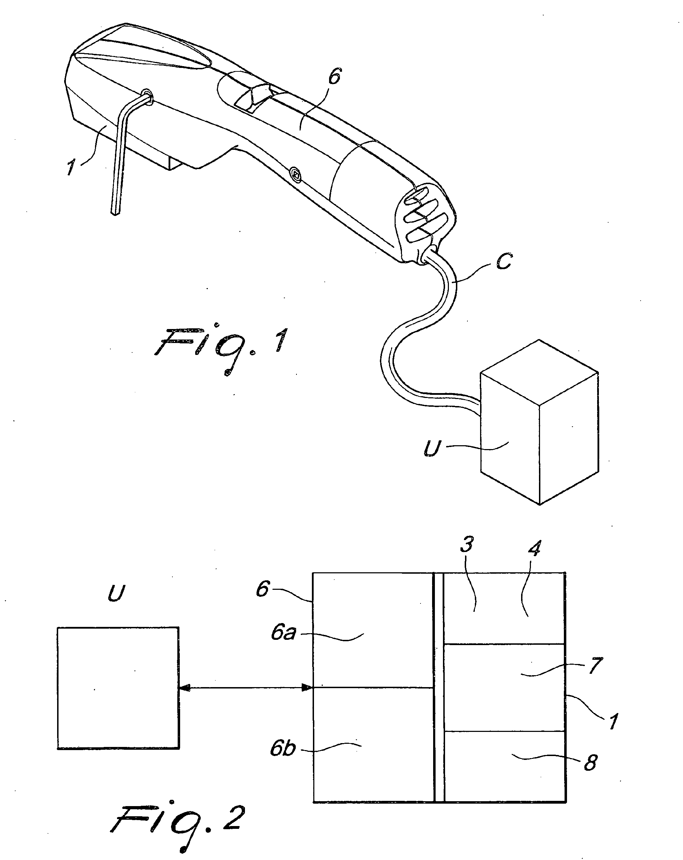

Figure 1 is a perspective view of a unit provided with a cartridge according to the invention;
Figure 2 is a functional block diagram of the unit and of the cartridge according to the invention;

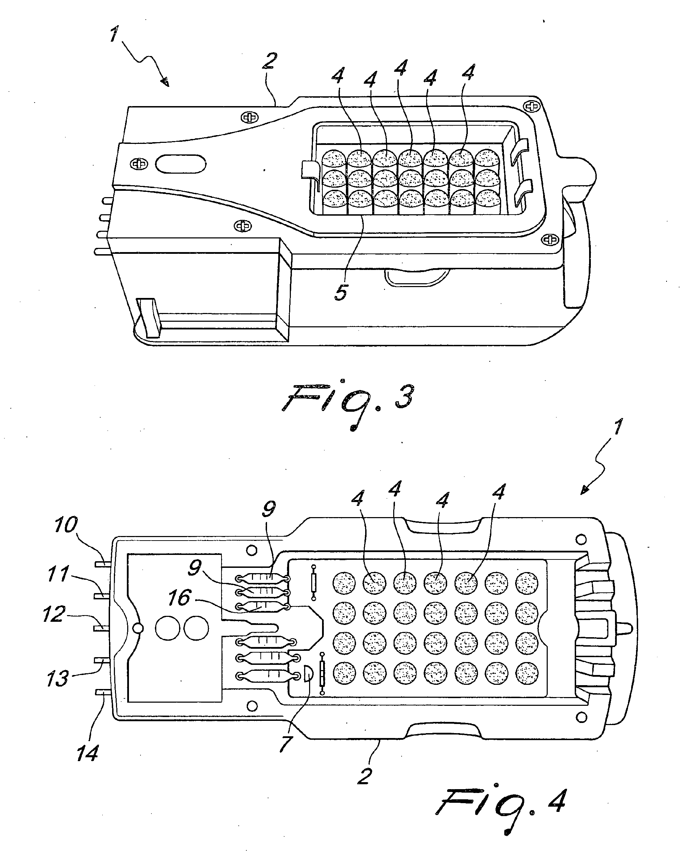

Figure 3 is a perspective view of a cartridge ;
Figure 4 is a top view of a cartridge;

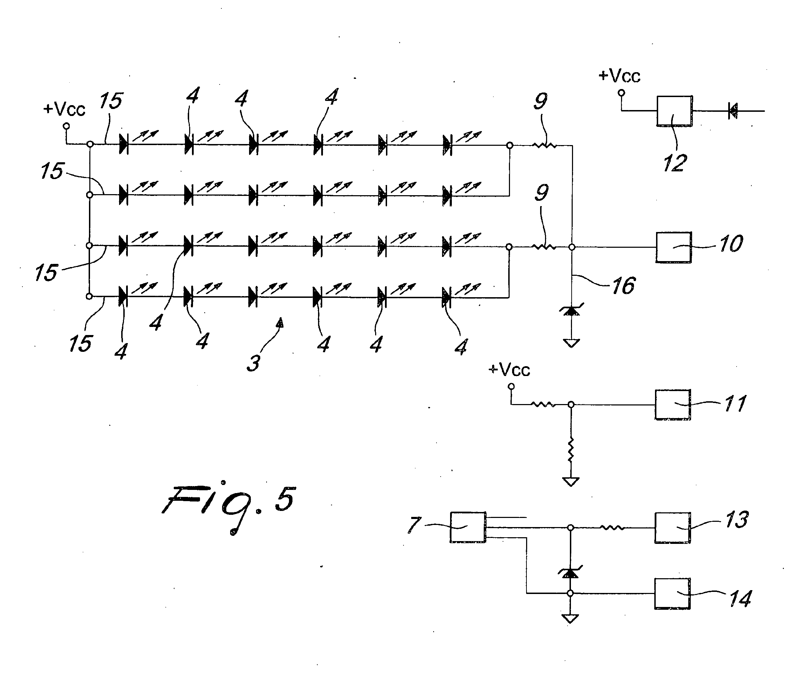

Figure 5 is a circuit diagram of a constructive embodiment of a cartridge.

With reference to the figures, the reference numeral 1 generally designates a skin treatment cartridge.

The cartridge 1 comprises a box-like body 2, which contains electrical and electronic circuits 3 for managing and controlling at least one light source, constituted by a light-emitting diodes 4 (hereinafter referenced simply by the acronym LED, Light Emitting Diode).

The body 2 is provided at the front with an opening 5 for the exit of the light beam; positively, the body 2 can be made of metallic material in order to have optimum heat dissipation; as an alternative, it is possible to insert surfaces provided with fins (dissipators) and in the handpiece 6 (through which the cartridge 1 receives electric power 6a) it is possible to arrange a forced ventilation apparatus 6b. The handpiece 6, in turn, is connected by means of a cable C to the control and management unit U.

Each LED 4, during use, faces the opening 5 and is therefore proximate to the skin of the user during treatment: the distance between the LED 4 and the skin is always constant, optimizing the effects of the treatment.

The LEDs 4 are a plurality and are distributed along multiple rows: in the embodiment described in the figure, the LEDs 4 are 28, distributed on four rows and seven columns, said columns being substantially parallel and perpendicular to the rows.

In the embodiment shown in the figure, the LEDs 4 are all arranged at the same distance from the surface of the skin to be treated, said distance being calculated as a function of the characteristics of the intensity and frequency of the beam emitted by the particular type of diode (LED 4).

Each LED 4 can in fact be adapted to emit a beam of light of a different color: treatment with a red beam has the purpose of stimulating the production of collagen by the derma, collagen being an essential protein required to repair damaged tissues and/or replace old tissues and further reducing postoperative swelling and pore size; treatment with a blue light beam is suitable for contrasting bacterial acne; treatment with a yellow light beam stimulates the lymphatic system and the nervous system, improving the tone of the skin.

Each cartridge 1 comprises a memory device 7 and a device 8 for recognition of the cartridge 1 on the part of the unit U through the handpiece 6 in which it is inserted.

The LEDs 4 are biased correctly by means of resistors 9 arranged in series thereto.

The cartridge is provided with electrical contacts for communication with the handpiece 6: a first connector 10 leads to the LEDs 4, a second connector 11 leads to a timing circuit, a third collector 12 for providing a high voltage level (+19 V in the embodiment shown in the figure), a fourth connector 13 leads to the memory unit 7, and the fifth and last connector 14 is the connector at the low voltage level (ground).

The LEDs 4 are arranged in series on respective branches 15, which are mutually in parallel with respect to the high voltage level (of the connector 12) and the time-controlled signal (of the connector 11), a branch 16 being derived toward the ground, before the respective connection to the time-controlled signal (of the connector 11), and a Zener diode 17 is connected thereon.

The operation of the invention is as follows: by arranging the cartridge 1 that is suitable for the treatment to which the user is to be subjected, it is possible to have a controlled beam of light emitted by the LEDs 4 which interacts with the derma, improving its appearance and facilitating the healing of certain diseases.

The cartridge 1 according to the invention can be fitted on the unit U disclosed in EP 1535582 in the name of the same Applicant and entitled "Light irradiation unit".

Positively, the surface to be treated is struck by a uniform light beam, which arrives from sources which are all equidistant from the skin, providing an optimum treatment even on small portions of the skin or on regions that are normally in shadow.

Advantageously, a same unit U can operate with different cartridges 1, for example having LEDs 4 with emissions in different colors.

It has thus been shown that the invention achieves the proposed aim and objects.

The invention thus conceived is susceptible of numerous modifications and variations, all of which are within the scope of the appended claims.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

The embodiment of the present invention shall be carried out in the most scrupulous compliance with the statutory and regulatory provisions related to the products of the invention or correlated thereto and following any required authorization of the corresponding competent authorities, with particular reference to regulations related to safety, environmental pollution and health.

In practice, the materials used, as well as the shapes and the dimensions, may be any according to requirements without thereby abandoning the scope of the protection of the appended claims.

## Claims

1. A radiation treatment unit for light radiation treatment of the skin of a person, comprising a control and management unit (U), a handpiece (6) and a plurality of different skin treatment cartridges (1), each of which being interchangeably insertable in said handpiece (6), said treatment cartridges (1) comprising each a light source, electrical contacts (10-14) for communication with said handpiece (6), a box-like body (2) which contains electrical and electronic circuits (3) for managing and controlling said light source, an opening (5) for the exit of the light beam, heat dissipation means, and a device (8) for recognition of the cartridge (1) by the control and management unit (U) through the handpiece in which it is insertable, **characterized in that**, each one of said treatment cartridges (1) further comprises a memory device (7), and a said light source constituted by a plurality of light-emitting diodes (4) distributed on multiple rows and of a type adapted to emit a beam of light of a colour suitable for a specific treatment, wherein each of said plurality of light-emitting diodes of a particular cartridge is adapted to emit a beam of light of the same colour, wherein said different treatment cartridges (1) have each light-emitting diodes (4) with an emission in different colour, each colour suitable for a specific treatment, wherein said light source (4) faces said opening (5) without any filter interposed, wherein all diodes of said plurality of light-emitting diodes of a respective cartridge are arranged to be during use at the
same distance from the surface of the skin to be treated, said distance being calculated as a function of the characteristics of the intensity and frequency of the beam emitted by the particular type of diode (4).

2. The radiation treatment unit according to claim 1, wherein said diodes (4) are at least four.

3. The radiation treatment unit according to claim 2, wherein said diodes (4) are 28 and are distributed along four substantially parallel rows and seven substantially parallel columns which are perpendicular to said rows.

4. The radiation treatment unit according to any of the preceding claims, wherein said diodes (4) emits a red beam of light in order to stimulate the production of collagen.

5. The radiation treatment unit according to any of the preceding claims, wherein said diodes (4) emit a blue beam of light in order to contrast bacterial acne.

6. The radiation treatment unit according to any of the preceding claims, wherein said diodes (4) are suitable to emit a yellow beam of light in order to stimulate the lymphatic system and the nervous system,

7. The radiation treatment unit according to any of the preceding claims, further comprising resistors (9) arranged in series to said diodes (4) which are correctly biased by means of said resistors (9).

8. The radiation treatment unit according to any of the preceding claims, wherein said diodes (4) are arranged in series on respective circuit branches (15), which are mutually parallel with respect to the high voltage level and the time-controlled signal, a circuit branch (16) being derived toward the ground before the respective connection to the time-controlled signal, on which a Zener diode is connected.

9. The radiation treatment unit according to any of the preceding claims, wherein said heat dissipation means are constituted by said body (2) being made of a metallic material or by fins provided on surfaces of said body (2).

## Patentansprüche

1. Bestrahlungsbehandlungseinheit für eine Lichtbestrahlungsbehandlung der Haut einer Person, eine Steuer- und Führungseinheit (U), ein Handstück (6) und mehrere verschiedene Hautbehandlungssteckmodule (1) umfassend, von denen jedes auswechselbar in das Handstück (6) einsetzbar ist, wobei die Behandlungssteckmodule (1) jeweils Folgendes umfassen: eine Lichtquelle, elektrische Kontakte (10 bis 14) zum Zusammenwirken mit dem Handstück (6), einen schachtelähnlichen Körper (2), der elektrische und elektronische Schaltungen (3) zum Führen und Steuern der Lichtquelle enthält, eine Öffnung (5) für den Austritt des Lichtstrahls, Wärmeableitungsmittel und eine Einrichtung (8) zum Erkennen des Steckmoduls (1) durch die Steuer- und Führungseinheit (U) durch das Handstück hindurch, in das es einsetzbar ist,
**dadurch gekennzeichnet, dass** jedes der Behandlungssteckmodule (1) ferner eine Speichereinrichtung (7) umfasst und die Lichtquelle von mehreren Leuchtdioden (4) gebildet wird, die in mehreren Reihen verteilt und dafür eingerichtet sind, einen Strahl Licht in einer Farbe auszustrahlen, die für eine spezielle Behandlung geeignet ist, wobei jede der mehreren Leuchtdioden eines bestimmten Steckmoduls dafür eingerichtet ist, einen Lichtstrahl der gleichen Farbe auszustrahlen, wobei die verschiedenen Behandlungssteckmodule (1) jeweils Leuchtdioden (4) mit einer Ausstrahlung in einer anderen Farbe aufweisen, wobei jede Farbe für eine spezielle Behandlung geeignet ist, wobei die Lichtquelle ohne einen zwischengeschobenen Filter der Öffnung (5) zugewandt ist, wobei alle Dioden der mehreren Leuchtdioden eines entsprechenden Steckmoduls angeordnet sind, dass sie während des Gebrauchs in gleichem Abstand von der zu behandelnden Haut liegen, wobei der Abstand als eine Funktion der Eigenschaften der Intensität und Frequenz des Strahles berechnet sind, der von der besonderen Art Diode (4) ausgestrahlt wird.

2. Bestrahlungsbehandlungseinheit nach Anspruch 1, wobei mindestens vier Dioden (4) vorhanden sind.

3. Bestrahlungsbehandlungseinheit nach Anspruch 2, wobei 28 Dioden (4) vorhanden sind, die in vier im Wesentlichen parallelen Reihen und sieben im Wesentlichen parallelen Spalten, die senkrecht zu den Reihen liegen, verteilt sind.

4. Bestrahlungsbehandlungseinheit nach einem der vorhergehenden Ansprüche, wobei die Dioden (4) einen roten Lichtstrahl ausstrahlen, um die Produktion von Collagen anzuregen.

5. Bestrahlungsbehandlungseinheit nach einem der vorhergehenden Ansprüche, wobei die Dioden (4) einen blauen Lichtstrahl ausstrahlen, um bakterieller Akne entgegenzuwirken.

6. Bestrahlungsbehandlungseinheit nach einem der vorhergehenden Ansprüche, wobei die Dioden (4) einen gelben Lichtstrahl ausstrahlen, um das Lymphsystem und das Nervensystem anzuregen.

7. Bestrahlungsbehandlungseinheit nach einem der vorhergehenden Ansprüche, ferner Widerstände (9) umfassend, die mit den Dioden (4) in Reihe geschaltet sind, die mit Hilfe der Widerstände (9) korrekt unter Vorspannung gesetzt werden.

8. Bestrahlungsbehandlungseinheit nach einem der vorhergehenden Ansprüche, wobei die Dioden (4) auf entsprechenden Stromkreisabzweigen (15) in Reihe angeordnet sind, die hinsichtlich des Hochspannungspegels und des zeitgesteuerten Signals zueinander parallel sind, wobei ein Stromkreisabzweig (16) vor der Verbindung mit dem zeitgesteuerten Signal, das mit einer Zener-Diode verbunden ist, zur Masse abgeleitet ist.

9. Bestrahlungsbehandlungseinheit nach einem der vorhergehenden Ansprüche, wobei die Wärmeableitungsmittel von dem Körper (2) gebildet sind, der aus einem Metallmaterial besteht, oder von Lamellen, die auf den Oberflächen des Körpers (2) bereitgestellt sind.

## Revendications

1. Unité de traitement avec des rayonnements, pour des traitements avec des rayonnements lumineux pour la peau d'une personne, comprenant une unité de commande et de gestion (U), une poignée (6) et une pluralité de cartouches différentes (1) pour le traitement de la peau, chacune desquelles pouvant être insérée de manière interchangeable dans ladite poignée (6), lesdites cartouches (1) comprenant chacune une source de lumière, des contacts électriques (10 - 14) de communication avec ladite poignée (6), un corps en forme de boîte (2) contenant les circuits électriques et électroniques (3) de gestion et de commande de ladite source de lumière, une ouverture (5) pour la sortie du rayon lumineux, des moyens appropriés de dissipation de la chaleur et un dispositif (8) pour la reconnaissance de la cartouche (1) par l'unité de commande et de gestion (U) à travers la poignée dans laquelle elle peut être insérée, **caractérisée en ce que** chacune desdites cartouches (1) comprend en outre un dispositif de mémoire (7), et ladite source de lumière constituée par une pluralité de diodes photoémettrices (4) distribuées sur de multiples rangées et d'un type prédisposé pour émettre un faisceau de lumière d'une couleur adaptée à un traitement spécifique, dans laquelle chacune desdites pluralités de diodes photoémettrices d'une cartouche particulière est adaptée pour émettre un rayon de lumière de la même couleur, dans laquelle lesdites cartouches différentes (1) ont chacune des diodes photoémettrices (4) avec émission de couleurs différentes, chaque couleur étant adaptée à un traitement spécifique, dans laquelle ladite source de lumière (4) est située face à ladite ouverture (5) sans aucun filtre interposé, dans laquelle toutes les diodes de ladite pluralité de diodes photoémettrices d'une cartouche respective sont disposées de manière à se trouver à une distance de la surface de la peau à traiter, ladite distance étant calculée en fonction des caractéristiques d'intensité et de fréquence du rayon émis par le type particulier de diode (4).

2. Unité de traitement avec des rayonnements selon la revendication 1, **caractérisée en ce que** lesdites diodes (4) sont au moins quatre.

3. Unité de traitement avec des rayonnements selon la revendication 2, **caractérisée en ce que** lesdites diodes (4) sont vingt-huit et sont distribuées sur quatre rangées sensiblement parallèles et sept colonnes sensiblement parallèles et orthogonales auxdites rangées.

4. Unité de traitement avec des rayonnements selon une des revendications précédentes, **caractérisée en ce que** lesdites diodes (4) émettent un faisceau de lumière de couleur rouge pour stimuler la production de collagène.

5. Unité de traitement avec des rayonnements selon une des revendications précédentes, **caractérisée en ce que** lesdites diodes (4) émettent un faisceau de lumière de couleur bleu pour lutter contre l'acné bactérienne.

6. Unité de traitement avec des rayonnements selon une des revendications précédentes, **caractérisée en ce que** lesdites diodes (4) sont adaptées pour l'émission d'un faisceau de lumière de couleur jaune pour stimuler le système lymphatique et le système nerveux.

7. Unité de traitement avec des rayonnements selon une des revendications précédentes comprenant en outre des résistances (9) placées en série avec lesdites diodes qui sont polarisées au moyen desdites résistances (9).

8. Unité de traitement avec des rayonnements selon une des revendications précédentes, **caractérisée en ce que** lesdites diodes (4) sont disposées en série sur des branches respectives de circuit (15), qui sont parallèles entre elles par rapport au niveau de haute tension et au signal temporisé, une branche de circuit (16) étant dérivée vers la masse avant la connexion respective au signal temporisé, sur laquelle est connectée une diode Zener.

9. Unité de traitement avec des rayonnements selon une des revendications précédentes, **caractérisée en ce que** lesdits moyens de dissipation de la chaleur sont constitués par ledit corps (2), qui est réalisé en matériau métallique ou avec des ailettes réalisées sur des surfaces dudit corps (2).
